# EUROPÄISCHE PATENTANMELDUNG

(11) **EP 2 169 575 A1**
(43) Veröffentlichungstag der Anmeldung: **31.03.2010**
(21) Anmeldenummer: 08016404.9
(22) Anmeldetag: 17.09.2008
(51) Int. Cl.: G06F 19/00

(54) **Automatische Überprüfung von Arzneimittelverordnungen für Patienten**

(71) Anmelder: Intercomponentware AG, 69190 Walldorf (DE)
(72) Erfinder: Karbach, Philipp, 69190 Walldorf (DE); Poß, Rudolf, 69190 Walldorf (DE); Sothmann, Sönke, 68309 Mannheim (DE)
(74) Vertreter: Müller-Boré & Partner Patentanwälte

(57) **Zusammenfassung**

Erfindung betrifft die Erstellung und/oder Optimierung eines medizinischen Verordnungsplans für zumindest einen Patienten, wobei ein erfindungsgemäßes Verfahren umfasst: Erfassen von primären Verordnungsdaten für den zumindest einen Patienten, wobei die primären Verordnungsdaten eine primäre Arzneimittelidentifikation umfassen; Festlegen eines Verblisterbarkeitsparameters für das zumindest eine Medikament. Dabei umfasst das Festlegen des Verblisterbarkeitsparameters: eine primäre Verfügbarkeitsprüfung zum Auswerten eines Verfügbarkeitsdatensatzes dahingehend, ob die primäre Arzneimittelidentifikation im Verfügbarkeitsdatensatz enthalten ist; und ein Zuweisen eines ersten Wertes zu dem Verblisterbarkeitsparameter oder eine Äquivalenzprüfung, je nachdem, ob die primäre Arzneimittelidentifikation im Verfügbarkeitsdatensatz enthalten ist oder nicht. Dabei umfasst die Äquivalenzprüfung: Ermitteln eines Äquivalenzdatensatzes aus einer Äquivalenzdatenbank derart, dass der Äquivalenzdatensatz Arzneimittelidentifikationen zumindest teilweise umfasst, die der primären Arzneimittelidentifikation durch die Äquivalenzdatenbank zugeordnet werden; eine sekundäre Verfügbarkeitsprüfung zum Auswerten des Verfügbarkeitsdatensatzen dahingehend, ob zumindest eine Arzneimittelidentifikation des Äquivalenzdatensatzes im Verfügbarkeitsdatensatz enthalten ist; und ein Zuweisen eines zweiten Wertes oder eines dritten Wertes zu dem Verblisterbarkeitsparameter, je nachdem ob falls zumindest eine Arzneimittelidentifikation des Äquivalenzdatensatzes im Verfügbarkeitsdatensatz enthalten ist oder nicht. Schließlich wird ein Verblisterbarkeitsindikator in Bezug auf die primäre Arzneimittelidentifikation und in Abhängigkeit vom zugewiesenen Wert des Verblisterbarkeitsparameters ausgegeben.

## Beschreibung

Die vorliegende Erfindung betrifft ein computer-implementiertes Verfahren, ein Computersystem und ein Computerprogrammprodukt zur automatischen Überprüfung bzw. Kontrolle von Arzneimittelverordnungen für einen Patienten.

Ältere Menschen insbesondere Bewohner eines Altenheimes erhalten oft eine Vielzahl von Medikamenten, die sie zum Teil in unterschiedlichen Verordnungen von wechselnden Ärzten verschrieben bekommen. Für die Bewohner gestaltet sich die Zuordnung der richtigen Medikamente zum jeweiligen Einnahmezeitpunkt als problematisch. Aufgrund fortgeschrittener Krankheiten oder altersbedingter Demenz ist ihnen oft nicht mehr zuverlässig möglich, die korrekte Stellung und Einnahme von Medikamenten einzuhalten. Daher wird das Stellen der Medikamente in Pflegeeinrichtungen von qualifizierten Pflegekräften übernommen. Gestellt bzw. in der richtigen Dosierung zur Einnahme vorbereitet werden Medikamente oft im Voraus für sieben Wochentage und vier Einnahmezeitpunkte (z.B. morgens, mittags, abends und nachts) je Wochentag (7x4-Schema). Für dieses 7x4-Schema gibt es auch vorgeformte Hilfen, sogenannten Dosetten, mit entsprechender Beschriftung. Für jeden Einnahmezeitpunkt ist ein Fach oder ein Döschen vorgesehen, in das die zu diesem Einnahmezeitpunkt einzunehmenden Medikamente von Hand einsortiert werden. Es gibt allerdings auch eine nicht unerhebliche Zahl von Patienten, für die aufgrund ihrer schweren Erkrankung das 7x4-Schema nicht anwendbar bzw. nicht ausreichend ist.

Für das korrekte Stellen der Medikamente werden genaue Angaben über den Namen des Medikaments oder eine sonstige eindeutige Identifikation des Medikaments, die Höhe der Dosierung und den Einnahmezeitpunkt benötigt. Diese Daten ergeben sich in der Regel aus der Verordnung durch einen Arztes und bilden insbesondere einen medizinischen Verordnungsplan. Dieser wird vorzugsweise nach einem geläufigen Schema angegeben, z.B. steht "Aspirin 1-1-1-0" für Aspirin jeweils morgens ("1" an der ersten Stelle der Zahlenreihe), mittags ("1" an der zweiten Stelle der Zahlenreihe) und abends ("1" an der dritten Stelle der Zahlenreihe) eine Tablette. Nachts ist keine Tablette verordnet ("0" an der vierten Stelle der Zahlenreihe). Die folgende Tabelle zeigt eine beispielhaften Wochenplan:

| Medikament | Mo | Di | Mi | Do | Fr | Sa | So |
|---|---|---|---|---|---|---|---|
| Delix® 5 plus | 1-0-½ -0 | 1-0-½ -0 | 1-0-½ -0 | 1-0-½ -0 | 1-0-½ -0 | 1-0-½ -0 | 1-0-½ -0 |
| Digimerck® minor 0,07mg Tbl. | 0-1-0-0 | | 0-1-0-0 | | 0-1-0-0 | | |
| isoket® retard 120mg Retardkaps. | 1-0-0-0 | 1-0-0-0 | 1-0-0-0 | 1-0-0-0 | 1-0-0-0 | 1-0-0-0 | 1-0-0-0 |

Dies hieße übersetzt in einem 7x4-Schema:

| | Mo | Di | Mi | Do | Fr | Sa | So |
|---|---|---|---|---|---|---|---|
| morgens | 1 Delix 1 isoket | 1 Delix 1 isoket | 1 Delix 1 isoket | 1 Delix 1 isoket | 1 Delix 1 isoket | 1 Delix 1 isoket | 1 Delix 1 isoket |
| mittags | 1 Digimerck | | 1 Digimerck | | 1 Digimerck | | |
| abends | ½ Delix | ½ Delix | ½ Delix | ½ Delix | ½ Delix | ½ Delix | ½ Delix |
| nachts | | | | | | | |

Im Durchschnitt erhalten Pflegeheimbewohner 6-7 Dauermedikamente und zusätzlich noch eine Akutmedikation. Das bedeutet, dass in Pflegeheimen für durchschnittlich 60-70 Patienten 6-7 Medikamente gestellt werden müssen. Dies geschieht in der Regel in der Spät- oder Nachtschicht, da hier der Aufwand für die Pflege geringer ist. Aufgrund der hohen Komplexität des Stellens ist der Prozess sehr zeitaufwändig und mitunter fehleranfällig. Einige Pflegeheime sind dazu übergegangen, das Stellen der Medikamente an die Apotheke abzugeben. Die Versorgung der Heime mit gestellten Arzneimitteln beispielsweise für eine Woche durch die Apotheke führt letztendlich zu einer Verlagerung des manuellen Stellens von Medikamenten in die Apotheke. Zunehmend gehen Apotheken dazu über, ihre Prozesse beim Stellen der Medikamente zu verbessern, z.B. indem Sie statt Dosetten im 7x4-Schema die Wochenration der Medikamente manuell verblistern, z.B. in sogenannten Kartenblistern.

Andere wiederum sahen die Notwendigkeit für eine Automatisierung der Prozesse mit speziell dafür entworfenen Sortiermaschinen, einer sogenannten Blistermaschine. Dies Blistermaschinen werden autark in den Apotheken aufgestellt und mit Medikamenten gefüllt. Dies geschieht mit kleinen Kassetten für jeweils ein Medikament. Die Kassetten verfügen über eine speziellen Dispenser, der die Medikamente auf Befehl herausfallen lässt. Im unteren Bereich der Maschine werden die herausgefallenen Medikamente in einem Tütchen aufgefangen und gemeinsam verschweißt. Durch die manuelle Eingabe der Verordnungspläne der Patienten weiß die Maschine welche Medikamente zu welchem Zeitpunkt herausfallen müssen, um gemeinsam verschweißt zu werden. Die einzelnen Tütchen werden vor dem Verschweißen von einem Drucker bedruckt.

Bei der individuellen Verblisterung von Arzneimitteln für einen Patienten werden verschiedene Medikamente unterschiedlicher Hersteller gemeinsam pro Dosierungszeitpunkt verpackt. Außerdem werden die Medikamente für einen Patienten oft von unterschiedlichen Ärzten verschrieben. Schließlich ist die Kapazität der bei der automatisierten Verblisterung eingesetzten Maschinen je nach Maschine beispielsweise auf einige Hundert verschiedene Medikamente begrenzt. All dies führt sehr schnell zu einer Grenze der Verlässlichkeit, Sicherheit und Flexibilität bei der individuellen Versorgung von Patienten mit Medikamenten.

Es ist Aufgabe der vorliegenden Erfindung, ein Verfahren, ein Computersystem und ein Computerprogrammprodukt bereitzustellen, die eine schnellere, zuverlässigere und insbesondere für die Benutzung durch mehrere Benutzer besonders flexible und überschaubare Versorgung von Patienten mit Medikamenten gewährleisten.

Diese Aufgabe wird durch ein computer-implementiertes Verfahren zur Erstellung und/oder Optimierung eines medizinischen Verordnungsplans mit den in Anspruch 1 angegebenen Merkmalen, ein computer-implementiertes Verfahren zur individuellen Verblisterung von Medikamenten mit den in Anspruch 12 angegebenen Merkmalen sowie ein Computersystem und ein Computerprogrammprodukt mit den in Ansprüchen 13 und 15 angegebenen Merkmalen gelöst. Bevorzugte Ausführungsformen sind Gegenstand der abhängigen Unteransprüche.

Somit stellt die Erfindung ein computer-implementiertes Verfahren zur automatisierten bzw. computergestützten Kontrolle bei der Erstellung und/oder Optimierung eines medizinischen Verordnungsplans für zumindest einen Patienten bereit, umfassend:
- Erfassen von primären Verordnungsdaten für den zumindest einen Patienten, wobei die primären Verordnungsdaten für zumindest eine Verordnung bzw. ein Medikament eine primäre Arzneimittelidentifikation umfassen bzw. festlegen;
- Festlegen bzw. Erstellen bzw. Ermitteln eines Verblisterbarkeitsparameters für das zumindest eine Medikament, wobei das Festlegen des Verblisterbarkeitsparameters umfasst:
   -- eine primäre Verfügbarkeitsprüfung zum Auswerten insbesondere Durchsuchen eines Verfügbarkeitsdatensatzes dahingehend, ob die primäre Arzneimittelidentifikation im Verfügbarkeitsdatensatz enthalten ist; und
   in Abhängigkeit vom Ergebnis der primären Verfügbarkeitsprüfung:
   -- Zuweisen eines ersten Wertes zu dem Verblisterbarkeitsparameter bzw. Setzen des Verblisterbarkeitsparameters auf einen ersten Wert, falls die primäre Arzneimittelidentifikation im Verfügbarkeitsdatensatz enthalten ist; und
   -- eine Äquivalenzprüfung, falls die primäre Arzneimittelidentifikation im Verfügbarkeitsdatensatz nicht enthalten ist, wobei die Äquivalenzprüfung umfasst:
      --- Ermitteln eines Äquivalenzdatensatzes aus einer Äquivalenzdatenbank derart, dass der Äquivalenzdatensatz Arzneimittelidentifikationen zumindest teilweise umfasst, die der primären Arzneimittelidentifikation durch die bzw. von der Äquivalenzdatenbank zugeordnet sind bzw. werden;
      --- eine sekundäre Verfügbarkeitsprüfung zum Auswerten des Verfügbarkeitsdatensatzen dahingehend, ob zumindest eine Arzneimittelidentifikation des Äquivalenzdatensatzes im Verfügbarkeitsdatensatz enthalten ist; und
         in Abhängigkeit vom Ergebnis der sekundären Verfügbarkeitsprüfung:
      --- Zuweisen eines zweiten Wertes zu dem Verblisterbarkeitsparameter bzw. Setzen des Verblisterbarkeitsparameters auf einen zweiten Wert, falls zumindest eine Arzneimittelidentifikation des Äquivalenzdatensatzes im Verfügbarkeitsdatensatz enthalten ist; und
      --- Zuweisen eines dritten Wertes zu dem Verblisterbarkeitsparameter bzw. Setzen des Verblisterbarkeitsparameters auf einen dritten Wert, falls keine Arzneimittelidentifikation des Äquivalenzdatensatzes im Verfügbarkeitsdatensatz enthalten ist; und
- Ausgeben eines Verblisterbarkeitsindikators in Bezug auf die primäre Arzneimittelidentifikation und in Abhängigkeit vom zugewiesenen Wert des Verblisterbarkeitsparameters.

Dadurch kann in automatisierter Weise sehr schnell, zuverlässig und vor allem übersichtlich und flexibel für jeden verantwortlichen Arzt zugänglich eine Verordnung als primäre Verordnung auf die Blisterbarkeit insbesondere durch eine Blistermaschine überprüft werden. So kann insbesondere nach Erfassen einer neu hinzugefügten Verordnung diese auf einem Übersichtsbildschirm angezeigt werden.

Vor den jeweiligen Medikamenten kann nun mit Hilfe des Verblisterbarkeitsindikators, beispielsweise in Form einer Ampeldarstellung darauf hingewiesen werden, welche Medikamente in eine automatisierte Blisterversorgung aufgenommen werden können. Beispielsweise steht ein grüner Punkt für die Möglichkeit der Verblisterung des eingegebenen Medikaments. Ein roter Punkt steht beispielsweise dafür, dass eine Verblisterung in diesem Fall ausgeschlossen ist. Dies wäre z.B. bei der Verschreibung von Tropfen der Fall. Ein beispielsweise gelber Punkt vor der zuletzt eingetragenen Verordnung könnte den verschreibenden Benutzer darauf hinweise, dass das gegenwärtig verschriebene Medikament durch zumindest einen generischen Austausch verblistert werden kann.

Grundlage für diese Darstellung mittels des Verblisterbarkeitsindikator ist die automatische Wertzuweisung zum Verblisterbarkeitsparameter. Die zugewiesenen Werte können beispielsweise logische und/oder numerische Werte umfassen: z.B. erster Wert entspricht logischem Wert "ja" oder "yes" oder numerischem Wert "1 ", zweiter Wert entspricht logischem Wert "vielleicht" oder "maybe" oder numerischem Wert "2", dritter Wert entspricht logischem Wert "nein" oder "no" oder numerischem Wert "3".

Dabei bietet die Erfindung eine effiziente und sehr flexible Zusammenführung mehrerer Datenbasen, nämlich zumindest dem zumindest einen Verfügbarkeitsdatensatz und der Äquivalenzdatenbank, welche unabhängig voneinander und insbesondere von verschiedenen Service-Anbietern in einem verteilten Netzwerk bereitgestellt und gewartet werden könnten. Dabei repräsentiert der Verfügbarkeitsdatensatz vorzugsweise eine spezifische Blistermaschine bzw. Blisteranlage und/oder eines spezifischen Blisterzentrums bzw. eines Blister-Serviceanbieters und wird entsprechend von diesem vorzugsweise online zur Verfügung gestellt. Die Äquivalenzdatenbank definiert für eine Vielzahl von Medikamenten generisch austauschbare Medikamente (Generika) und kann beispielsweise von einer Behörde, wie etwa einem Amt, einem Ministerium oder einer Krankenkasse vorzugsweise online zur Verfügung gestellt werden. In einer anderen bevorzugen Ausführungsform werden diese Datenbasen lokal abgelegt. Sie könnten dabei regelmäßig aktualisiert werden.

Vorzugsweise stellt eine Arzneimittelidentifikation eine eindeutige Kennung in Form einer Buchstaben- und/oder Ziffernfolge dar. Beispielsweise umfasst eine Arzneimittelidentifikation eine Pharmazentralnummer (PZN), wie sie in Deutschland üblich ist. Alternativ oder zusätzlich könnte die Arzneimittelidentifikation auch andere lokal, regional, national und/oder global übliche oder normierte Kennungen bzw. Identifikationsnummer bzw. -zeichen umfassen, wie z.B. M2.

Auf der Medikamentenebene wird bei einem gegebenen Medikament vorzugsweise die dazugehörige PZN mit dem Verfügbarkeitsdatensatz (Positivliste) des Blisterzentrums verglichen. Die Positivliste kann dabei im System vorhanden sein, oder dynamisch angebunden werden, z.B. über einen Webservice aus dem Blisterzentrum oder einen SOA (Serviceorientierte Architektur)-basierten Webservice. Für den Fall, dass die gegebene PZN in der Positivliste des Blisterzentrums vorhanden ist, wird als Ergebnis der erste Wert (z.B. "YES") zurückgegeben. Beispielsweise könnte eine primäre Verordnung des Patienten ASS 100 Hexal (PZN 7402204, PZN 7402210) enthalten, welches in der Positivliste des Blisterzentrums enthalten ist.

Für den Fall, dass die gegebenen PZN in der Positivliste des Blisterzentrums nicht vorhanden ist, wird als weitere Datenbasis die Äquivalenzdatenbank herangezogen. Diese ist vorzugsweise als sogenannte aut-idem-Liste ausgebildet. Nun werden aus der Äquivalezdatenbank, welche eine Liste der generisch austauschbaren Medikamente (z.B. der aut-idem-Liste) umfasst, diejenigen Medikamente herausgefiltet, die mit dem gegebenen Medikament generisch austauschbar sind die zugehörige PZN zurückgegeben. Die aut-idem-Liste wird lokal hinterlegt oder dynamisch angebunden, z.B. über einen Webservice eines Serviceanbieters zur Verfügung gestellt. Vorzugsweise wird die Äquivalezdatenbank über einen SOAbasierten Webservice bereitgestellt. Die erhaltene PZN der generisch austauschbaren Medikamente werden nun mit der Positivliste des Blisterzentrums abgeglichen. Sollte sich eines oder mehrere der Medikamente auf der Positivliste des Blisterzentrums wieder finden, wird der Verblisterbarkeitsparameter auf den zweiten Wert (z.B. "MAYBE") gesetzt. Vorzugsweise werden die entsprechenden PZN in einem Austauschdatensatz als sekundäre Arzneimittelidentifikationen zusammengefasst. Beispielsweise enthalten die primären Verordnungsdaten des Patienten ASS 1A Pharma (PZN 8612406 und PZN 8612412), während das Blisterzentrum hierzu nur das äquivalente Medikament der Firma Hexal (PZN 7402204, PZN 7402210) führt.

Für den Fall dass weder die gegebene PZN, noch die PZN generisch austauschbarer Medikamente in der Positivliste des Blisterzentrums enthalten sind, wird dem Verblisterbarkeitsparameter der dritte Wert (z.B. "NO") zugewiesen. Als Beispiel enthalten die primären Verordnungsdaten des Patienten Atosil Tropfen (PZN 6875030 und PZN 84913), die nicht in der Liste des Blisterzentrums enthalten sind (flüssige Darreichungsform).

Nun hat der Benutzer vorzugsweise die Möglichkeit durch einen Blick auf den angezeigten medizinischen Verordnungsplan (Medikationsplan) schnell zu erfassen, wie viele von den Medikamenten verblistert werden können und ob evtl. durch den generischen Austausch mehr Medikamente mit in die Blisterversorgung mit aufgenommen werden können.

Vorzugsweise umfasst die sekundäre Verfügbarkeitsprüfung ein Erstellen eines Austauschdatensatzes, welcher sekundäre Arzneimittelidentifikationen festlegt bzw. definiert bzw. umfasst, die sowohl im Äquivalenzdatensatz als auch im Verfügbarkeitsdatensatz enthalten sind, und wobei das Verfahren im Fall, dass dem Verblisterbarkeitsparameter in Bezug auf das zumindest eine Medikament der zweite Wert zugewiesen wurde, umfasst:
- Erfassen einer Austauschabfrage durch eine Benutzereingabe; und
- Ausgeben von Repräsentationen der sekundären Arzneimittelidentifikationen, d.h. Ausgeben von sekundären Arzneimittelrepräsentationen bzw. einer Repräsentation für jede sekundäre Arzneimittelidentifikation bzw. der vom Austauschdatensatz festgelegten Arzneimittelidentifikationen, in Bezug auf das zumindest eine Medikament.

Beispielsweise durch einen Mausklick auf den Verblisterbarkeitsindikator (z.B. den gelben Farbpunkt vor der Verordnung) sieht der Benutzer in einem neuen Fenster eine Liste der Medikamente, die sich für den generischen Austausch eignen und in der Positivliste des Blisterzentrums enthalten sind. Vorzugsweise kann der Benutzer beispielsweise durch einen Mausklick auf das gewünschte Austauschmedikament dieses ad-hoc in die Verordnung aufnehmen und damit das primäre (nicht verblisterbare) Medikament ersetzen.

Vorzugsweise umfasst das Verfahren außerdem:
- Erfassen einer Auswahl, insbesondere in Form einer Benutzereingabe, einer sekundären Arzneimittelidentifikation aus dem Austauschdatensatz; und
- Erstellen von sekundären Verordnungsdaten aus den primären Verordnungsdaten umfassend ein insbesondere automatisches Ersetzen der primären Arzneimittelidentifikation durch die sekundäre Arzneimittelidentifikation.

Vorzugsweise umfassen die primären Verordnungsdaten für das zumindest eine Medikament primäre Dosierungsdaten und das Erstellen der sekundären Verordnungsdaten umfasst vorzugsweise:
- Ermitteln von sekundären Dosierungsdaten für ein durch die sekundäre Arzneimittelidentifikation festgelegtes Austauschmedikament aus der Äquivalenzdatenbank in Abhängigkeit von den primären Dosierungsdaten; und
- insbesondere automatisches Ersetzen der primären Dosierungsdaten durch die sekundären Dosierungsdaten.

Dies ermöglicht eine besondere schnelle und vor allem sichere Erstellung bzw. Optimierung eines medizinischen Verordnungsplans. Dabei ist es beim Ersetzen eines ursprünglich bzw. primär verordneten Medikaments durch ein gleich wirkendes bzw. äquivalentes Austauschmedikament nicht mehr erforderlich, eine aufgrund einer anderen Darreichungsform oder Konzentration desselben Wirkstoffs im Austauschmedikament oder einer anderen Wirksamkeit eines eventuell anderen Wirkstoffs im Austauschmedikament erforderliche Anpassung der Dosierung manuell vorzunehmen. Statt dessen erfolgt diese Anpassung vorzugsweise automatisiert auf Grundlage von Umrechnungsdaten, welche über die vorzugsweise zentrale und konsolidierte Äquivalenzdatenbank zur Verfügung gestellt werden. Damit erfolgt die Erstellung bzw. Optimierung des medizinischen Verordnungsplans deutlich schneller und sicherer.

Vorzugsweise umfasst das Verfahren:
- Erfassen einer Patientenidentifikation für den zumindest einen Patienten; und
- Abrufen von gespeicherten Patientendaten aus einer insbesondere zentralen Patientendatenbank in Abhängigkeit von der Patientenidentifikation.

Besonders bevorzugt umfasst das Erfassen von primären Verordnungsdaten für den zumindest einen Patienten ein Abrufen der primären Verordnungsdaten für den zumindest einen Patienten aus der Patientendatenbank in Abhängigkeit von der Patientenidentifikation.

Vorzugsweise umfasst das Verfahren ein Bereitstellen einer Kompatibilitätsdatenbank, in der einer Vielzahl von Arzneimittelidentifikationen eine oder mehrere andere Arzneimittelidentifikationen als inkompatibel zugeordnet werden,
wobei die gespeicherten Patientendaten konsolidierte Verordnungsdaten umfassen, welche jeweils zumindest eine konsolidierte Arzneimittelidentifikation umfassen und
wobei die Äquivalenzprüfung eine Kompatibilitätsprüfung derart umfasst, dass der Äquivalenzdatensatz keine Arzneimittelidentifikationen umfasst, die zumindest einer der konsolidierten Arzneimittelidentifikationenen durch die Kompatibilitätsdatenbank (evtl. zusammen mit der Äquivalenzdatenbank implementiert) als inkompatibel zugeordnet werden bzw. sind. Damit wird insbesondere der Patient automatisch vor unerwünschten Wechselwirkungen verschiedener Verordnungen geschützt.

Vorzugsweise umfassen die gespeicherten Patientendaten einen individuellen bzw. patientenspezifischen Akzeptanzdatensatz des zumindest einen Patienten festlegen bzw. umfassen, welcher eine oder mehrere Arzneimittelidentifikationen als von dem zumindest einen Patienten nicht akzeptiert festlegt bzw. definiert, und wobei die Äquivalenzprüfung eine Akzeptanzprüfung derart umfasst, dass der Äquivalenzdatensatz keine Arzneimittelidentifikation umfasst, die vom Akzeptanzdatensatz als von dem zumindest einen Patienten nicht akzeptiert festgelegt ist. Damit können in besonders effizienter Weise automatiert und zuverlässig individuelle Unverträglichkeiten und/oder persönlichen Abneigungen des Patienten berücksichtigt werden.

Vorzugsweise umfassen die primären Verordnungsdaten für eine Vielzahl von Medikamenten jeweils eine primäre Arzneimittelidentifikationen, wobei das Festlegen eines Verblisterbarkeitsparameters für eine Vielzahl, insbesondere jedes dieser Medikamente erfolgt. Besonders bevorzugt umfasst das Verfahren ein Zuweisen eines Effizienzwerts zu dem Verfügbarkeitsdatensatz, wobei das Zuweisen des Effizienzwerts umfasst:
- Bereitstellen einer Effizienzwertrelation bzw. Effizienzwertskala, die jedem Wert des Verblisterbarkeitsparameters einen Effizienzgewichtungswert zuordnet;
- Ermitteln des Effizienzwerts in Abhängikeit von den Effizienzgewichtungswerten aller zu diesem Verfügbarkeitsdatensatz für die einzelnen Medikamente festgelegten Verblisterbarkeitsparameter. Damit kann besonders schnell und einfach eine zuverlässige Aussage über den Umfang bzw. Grad der Verblisterbarkeit für einen Patienten getroffen werden.

Besonders bevorzugt umfasst das Verfahren ein
- Bereitstellen einer Vielzahl von Verfügbarkeitsdatensätzen, denen jeweils ein Auftragsdatensatz zugeordnet ist, wobei das Zuweisen eines Effizienzwerts für jeden der Verfügbarkeitsdatensätze erfolgt;
- Ermitteln eines optimalen Verfügbarkeitsdatensatzes aus der Vielzahl von Verfügbarkeitsdatensätzen auf Basis der ihnen zugewiesenen Effizienzwerte; und
- Zuordnen des dem optimalen Verfügbarkeitsdatensatz zugeordneten Auftragsdatensatzes zu Patientendaten insbesondere zu dem medizinischen Verordnungsplan des zumindest einen Patienten. Damit kann automatisch eine Auswahl auf einer Vielzahl möglicher Blisterzentren gefunden werden, bei dem der jeweilige Patient am besten mit Blistern versorgt werden kann. Die entsprechenden Auftragsdaten (z.B. Kontaktdaten) könnten automatisch hinterlegt bzw. dem Patienten zugeordnet werden.

Außerdem stellt die Erfindung ein Computer-implementiertes Verfahren zur individuellen Verblisterung von Medikamenten für zumindest einen Patienten bereit, welches umfasst:
- ein Verfahren zur Erstellung und/oder Optimierung eines medizinischen Verordnungsplans nach Anspruch 3, 4 oder einem der Ansprüche 5 bis 11, soweit er auf Anspruch 3 rückbezogen ist;
- Ermitteln eines dem Patienten zugeordneten Auftragsdatensatzes;
- Ermitteln von dem Patienten zugeordneten Versorgungsintervalldaten;
- Übermitteln der sekundären Verordnungsdaten zusammen mit den Versorgungsintervalldaten an eine durch den Auftragsdatensatz festgelegte, automatisch gesteuerte Verblisterungsanlage; und
- automatisches Verblistern von Medikamenten gemäß den sekundären Verordnungsdaten für einen in den Versorgungsintervalldaten festgelegten Versorgungszeitraum.

Außerdem stellt die Erfindung ein Computersystem zur Erstellung und/oder Optimierung eines medizinischen Verordnungsplans für zumindest einen Patienten bereit, welches umfasst:
- eine Eingabeschnittstelle zum Erfassen von primären Verordnungsdaten für den zumindest einen Patienten, wobei die primären Verordnungsdaten für zumindest ein Medikament eine primäre Arzneimittelidentifikation umfassen;
- ein Analysemodul, welches ausgelegt ist zum Festlegen eines Verblisterbarkeitsparameters für das zumindest eine Medikament, wobei das Festlegen des Verblisterbarkeitsparameters umfasst:
   -- eine primäre Verfügbarkeitsprüfung zum Auswerten eines Verfügbarkeitsdatensatzes dahingehend, ob die primäre Arzneimittelidentifikation im Verfügbarkeitsdatensatz enthalten ist; und in Abhängigkeit vom Ergebnis der primären Verfügbarkeitsprüfung:
   -- Zuweisen eines ersten Wertes zu dem Verblisterbarkeitsparameter, falls die primäre Arzneimittelidentifikation im Verfügbarkeitsdatensatz enthalten ist; und
   -- eine Äquivalenzprüfung, falls die primäre Arzneimittelidentifikation im Verfügbarkeitsdatensatz nicht enthalten sind, wobei die Äquivalenzprüfung umfasst:
      --- Ermitteln eines Äquivalenzdatensatzes aus einer Äquivalenzdatenbank derart, dass der Äquivalenzdatensatz Arzneimittelidentifikationen zumindest teilweise umfasst, die der primären Arzneimittelidentifikation durch die Äquivalenzdatenbank zugeordnet werden;
      --- eine sekundäre Verfügbarkeitsprüfung zum Auswerten des Verfügbarkeitsdatensatzen dahingehend, ob zumindest eine Arzneimittelidentifikation des Äquivalenzdatensatzes im Verfügbarkeitsdatensatz enthalten ist; und
         in Abhängigkeit vom Ergebnis der sekundären Verfügbarkeitsprüfung:
      --- Zuweisen eines zweiten Wertes zu dem Verblisterbarkeitsparameter, falls zumindest eine Arzneimittelidentifikation des Äquivalenzdatensatzes im Verfügbarkeitsdatensatz enthalten ist; und
      --- Zuweisen eines dritten Wertes zu dem Verblisterbarkeitsparameter, falls keine Arzneimittelidentifikation des Äquivalenzdatensatzes im Verfügbarkeitsdatensatz enthalten ist; und
- eines Ausgabeeinheit zum Ausgeben eines Verblisterbarkeitsindikators in Bezug auf die primäre Arzneimittelidentifikation und in Abhängigkeit vom zugewiesenen Wert des Verblisterbarkeitsparameters.

Vorzugsweise umfasst das Computersystem eine Identifikationsdatenzuordnungseinheit zum Übersetzung zwischen einem normierten Identifikationscode von Medikamenten, wie z.B. der Arzneimittelidentifikation insbesondere in Form der Pharmazentralnummer (PZN) und einer Vertriebsbezeichnung/einem Vertriebsnamen, Markenbezeichnung, Handelsname, oder einer Kombination aus dem Hersteller und dem Wirkstoff.

Vorzugsweise ist das Computersystem ausgelegt, ein Verfahren gemäß der vorliegenden Erfindung in einer oder mehreren der beschriebenen Ausführungsformen auszuführen.

Die Erfindung bietet außerdem ein Computerprogrammprodukt, insbesondere in Form eines maschinenlesbaren Mediums, als Signal und/oder als Datenstrom ausgestaltet, welches maschinenlesbaren Programmcode umfasst, der, wenn er geladen wird auf einem Computer, zur Ausführung eines Verfahrens gemäß der vorliegenden Erfindung in einer oder mehreren der beschriebenen Ausführungsformen führt bzw. geeignet ist.

In einem weiteren Aspekt betrifft die Erfindung damit vorzugsweise ein computer-implementiertes Verfahren zur individuellen Verblisterung von Medikamenten für zumindest einen Patienten. Dieses Verfahren umfasst dabei vorzugsweise ein computer-implementiertes Verfahren zur Erstellung und/oder Optimierung eines medizinischen Verordnungsplans für den zumindest einen Patienten gemäß der vorliegenden Erfindung insbesondere in einer der beschriebenen bevorzugten Ausführungsformen.

Die Erfindung wird nachfolgend anhand bevorzugter Ausführungsformen mit Bezug auf die begleitende Zeichnungen beschrieben. Dabei zeigen:
- Fig.1: eine schematische Darstellung eines Verfahrens gemäß einer bevorzugten Ausführungsform der vorliegenden Erfindung;
- Fig. 2: eine graphische Benutzeroberfläche gemäß einer gevorzugten Ausführungsform eines erfindungsgemäßen Systems; und
- Fig.3: zeigt ein schematisches Datenmodell der in einer bevorzugten Ausführungsform der vorliegenden Erfindung miteinander vernetzen Datenbasen.
- Fig. 4: eine schematische Darstellung eines beispielhaften Systems zum Implementieren der Erfindung

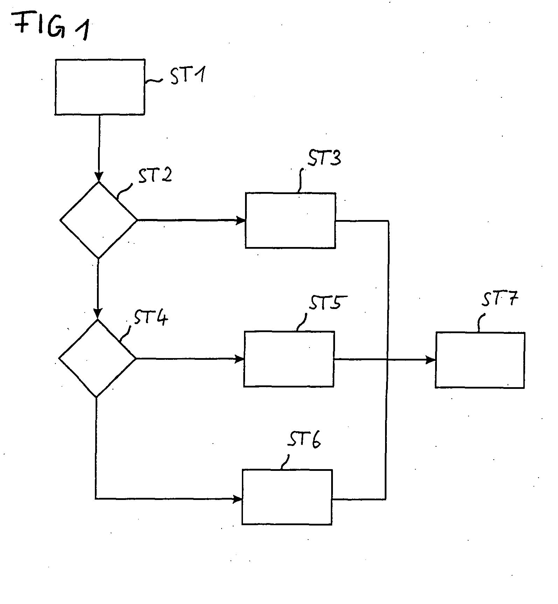

**Fig. 1** zeigt eine vereinfachte schematische Darstellung zur Verdeutlichung eines Verfahrens gemäß einer bevorzugten Ausführungsform der vorliegenden Erfindung. Dabei umfasst ein erster Schritt ST1 ein Erfassen von primären Verordnungsdaten für zumindest einen Patienten. Dabei werden die primären Verordnungsdaten vorzugsweise entweder unmittelbar vom Benutzer, wie z.B. einem Arzt insbesondere mittels einer graphischen Benutzeroberfläche einer entsprechenden Benutzerschnittstelle in ein Computersystem eingegeben oder aus einer insbesondere zentralen Patientendatenbank abgerufen. Die Abfrage der Patientendaten erfolgt dabei beispielsweise in Reaktion auf eine Eingabe von Patientenidentifikationsdaten bzw. einer Patientenidentifikation durch den Benutzer. Die Eingabe von Verordnungsdaten und/oder einer Patientenidentifikation kann beispielsweise über einen Desktop-Computer in der Artzpraxis oder einem Pflegeheim, aber auch über einen tragbaren Computer oder ein anderes geeignetes, mobiles Eingabegerät während eines Hausbesuchs des Arztes beim Patienten oder während eines Besuches des Arztes im Pflegeheim des Patienten erfolgen. Dazu bildet oder umfasst der jeweilige Computer bzw. das Eingabegerät vorzugsweise eine Eingabeschnittstelle eines erfindungsgemäßen Computersystems.

In einer weiteren bevorzugten Ausgestaltung erfolgt die Eingabe einer Patientenidentifikation durch ein geeignetes Eingabegerät in einer Apotheke, welche einen Auftrag zur Versorgung des Patienten mit Arzneimitteln gemäß einem dem Patienten in der zentralen Patientendatenbank zugeordneten medizinischen Verordnungsplan ausführt. Für eine effiziente und vor allem sichere Bearbeitung bzw. Optimierung bzw. Überprüfung eines medizinischen Verordnungsplans und eine sichere und fehlerfrei Versorgung des Patienten mit entsprechenden Medikamenten ist ein erfindungsgemäßes Verfahren besonders hilfreich. Dabei werden Schritt ST1 insbesondere in Reaktion auf die Eingabe einer Patientenidentifikation primäre Verordnungsdaten für den Patienten von einer zentralen Patientendatenbank abgerufen.

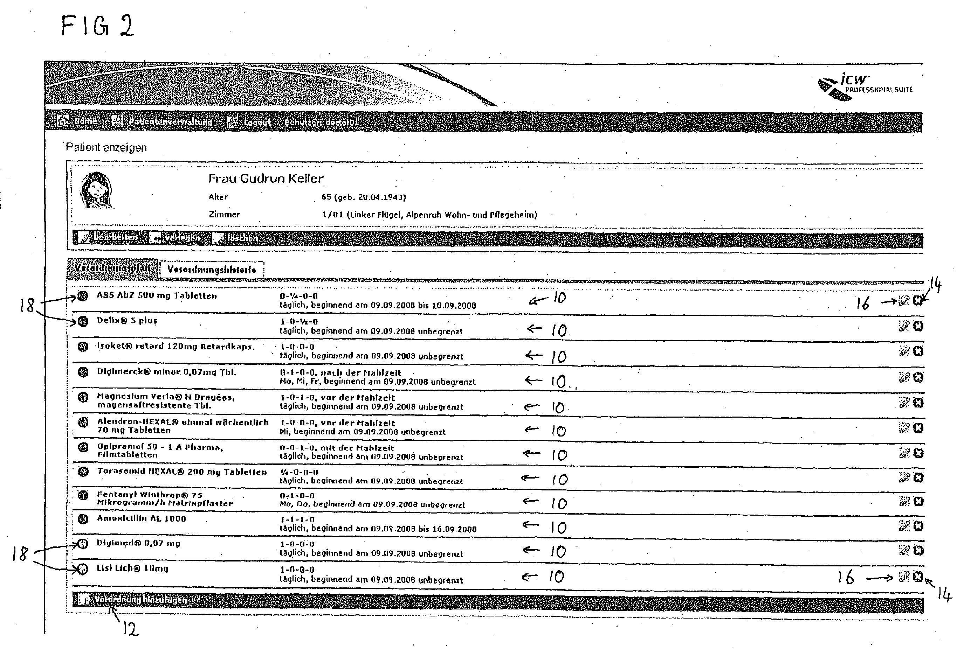

**Fig. 2** zeigt eine graphische Benutzeroberfläche einer Eingabeschnittstelle und/oder Ausgabeeinheit gemäß einer bevorzugte Ausführungsform der vorliegenden Erfindung. Dabei wird dem Benutzer, z.B. dem Arzt ein medizinischer Verordnungsplan einer Patientin mit einer Vielzahl von Verordnungen 10 in einer Liste, z.B. in einzelnen Reihen, präsentiert. Durch Betätigen (z.B. per Mausklick) eines Eingabefeldes 12 kann der Benutzer eine weitere Verordnung hinzufügen, durch Betätigen entsprechender "Löschen"-Felder 14 wieder aus dem Verordnungsplan entfernen. Durch Betätigen von Editierfeldern 16 kann er eine vorbestimmte bzw. vorbestimmbare (bevorzugt jede) Verordnung einzeln oder zusammen mit einer oder mehreren weiteren vorbestimmten bzw. vorbestimmbaren Verordnungen editieren. Die in Fig. 2 dargestellten Verordnungsdaten 10 könnten primäre, d.h. insbesondere noch nicht (bevorzugt automatisch) geänderte bzw. ersetzte Verordnungsdaten sein. Vorzugsweise umfassen die primären Verordnungsdaten zumindest eine primäre Arzneimittelidentifikation. Diese ist in Fig. 2 nicht dargestellt, sondern wird vorzugsweise erst beim Editieren einzelner Verordnungsdaten bzw. Verordnungen für den Benutzer sichtbar bzw. direkt zugänglich. Ansonsten werden die Arzneimittelidentifikation insbesondere als interne Parametern genutzt.

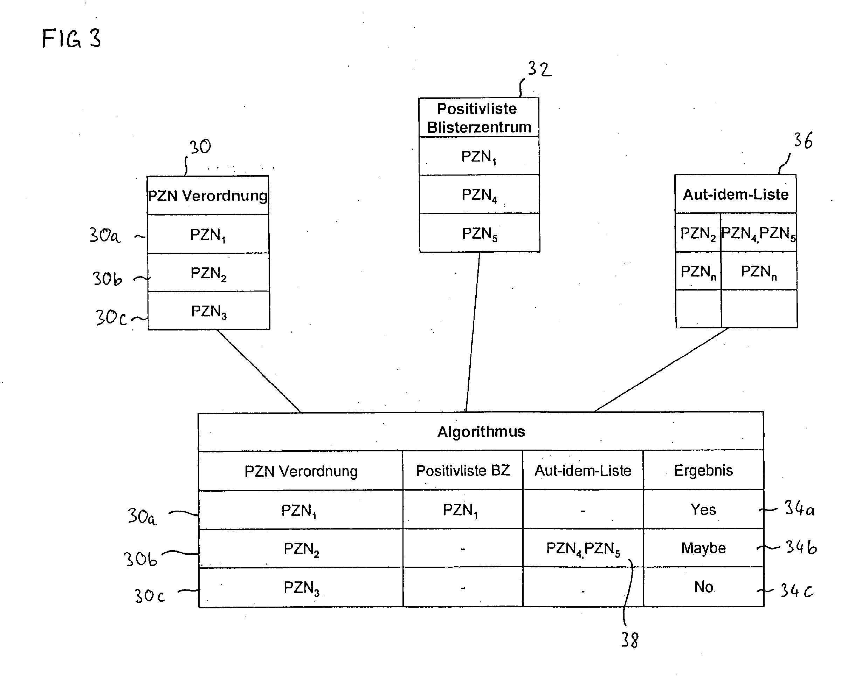

**Fig.3** zeigt ein schematisches Datenmodell der in einer bevorzugten Ausführungsform der vorliegenden Erfindung miteinander vernetzen Datenbasen. Insbesondere umfassen diese Datenbasen den Verordnungsplan 30 eines Patienten, wobei der Verordnungsplan 30 für eine Vielzahl von Verordnungen 30a, 30b, 30c Arzneimittelidentifikationen (insbesondere Pharmazentralnummern) PZN₁, PZN₂, PZN₃ umfasst bzw. zuordnet.

Auf Basis dieses Verordnungsplans 30 wird nun vorzugsweise intern und vorzugsweise für jedes Medikament bzw. jede Verordnung ein Verblisterbarkeitsparameter festgelegt. Dafür wird zunächst ein Verfügbarkeitsdatensatz 32, beispielsweise in Form einer Positivliste eines Blisterzentrums, bereitgestellt bzw. ermittelt, der ein Vielzahl von Arzneimittelidentifikationen PZN₁, PZN₄, PZN₅ von verblisterbaren Medikamenten umfasst bzw. festlegt. Damit wird eine primäre Verfügbarkeitsprüfung ST2 durchgeführt. Insbesondere wird dabei der Verfügbarkeitsdatensatz 32, dahingehend ausgewertet, ob die primären Arzneimittelidentifikationen PZN₁, PZN₂, PZN₃ im Verfügbarkeitsdatensatz enthalten sind. In der in Fig. 3 gezeigten Ausführungsform trifft dies für die erstgenannte primäre Verordnung PZN₁ zu, weshalb in einem Schritt ST3 dem Verblisterbarkeitsparameter 34a der ersten Verordnung 30a der erste Wert, im gezeigten Beispiel der Wert "Yes" zugewiesen wird.

Für die andern beiden schematisch gezeigten Verordnungen 30b, 30c verläuft die Verfügbarkeitsprüfung negativ. Somit wird für diese beiden Verordnungen in einem Schritt ST4 eine Äquivalenzprüfung durchgeführt. Dazu wird auf eine Äquivalenzdatenbank 36 zugegriffen. Die Äquivalenzdatenbank 36 ist ausgelegt, einer Vielzahl von Medikamenten äquivalente bzw. gleichwirkende bzw. generische Medikamente zuzuordnen. Dies erfolgt insbesondere auf Basis der Arzneimittelidentifikation(en) dieser Medikamente. So ordnet die gezeigte Äquivalenzdatenbank 36 beispielsweise der PZN₂ die beiden PZN₄ und PZN₅ zu. Dementsprechend wird im Schritt ST4 der Äquivalenzprüfung für die zweite primäre Verordnung 30b ein Äquivalenzdatensatz 38 ermittelt, welcher eben diese beiden Arzneimittelidentifikationen PZN₄ und PZN₅ umfasst. In einer sekundären Verfügbarkeitsprüfung wird nun die Positivliste 32 dahingehend ausgewertet, ob zumindest eine Arzneimittelidentifikation des Äquivalenzdatensatzes 38 in der Positivliste 32 enthalten ist. Im gezeigten Beispiel trifft dies für beide Arzneimittelidentifikationen des Äquivalenzdatensatzes 38 zu.

Damit wird nun in einem Schritt ST5 dem Verblisterbarkeitsparameter 34b der zweiten primären Verordnung 30b der zweite Wert (hier "Maybe") zugewiesen. Für die dritte primäre Verordnung 30c könnte im Schritt ST4 keine äquivalente Arzneimittelidentifikation ermittelt werden. Damit verlief auch die sekundäre Verfügbarkeitsprüfung negativ, weshalb dem Verblisterbarkeitsparameter 34c der dritten primären Verordnung 30c der dritte Wert (hier "No") zugewiesen wird.

Wie in Fig. 1 gezeigt wird nun in einem Schritt ST7 in Abhängigkeit vom Wert des jeweiligen Verblisterbarkeitsparameters ein Verblisterbarkeitsindikator ausgegeben, der dem Benutzer zumindest teilweise eine Unterscheidung zwischen den verschiedenen ermittelten Werten der Verblisterbarkeitsparameter erlaubt. Fig. 2 zeigt bevorzugte Verblisterbarkeitsindikatoren 18 in Form von farbigen Punkten (insbesondere in Art einer Ampel und/oder in Ampelfarben). Dabei wird vorzugsweise je nach Wert des Verblisterbarkeitsparameters eine von drei möglichen Farben ausgewählt. Vorzugsweise sind diese Verblisterbarkeitsindikatoren durch einen Benutzer betätigbar bzw. aktivierbar, wobei besonders bevorzugt bei Betätigung (z.B. Mausklick) eines Indikators, der den zweiten Wert des Verblisterbarkeitsparameters repräsentiert dem Benutzer ein Austauschdatensatz insbesondere als Liste von Medikamenten, deren Arzneimittelidentifikation sowohl im Äquivalenzdatensatz als auch in der Positivliste enthalten ist, zur Auswahl angeboten werden. Beispielsweise durch Mausklick auf das gewünschte Medikament (bzw. durch eine beliebeige andere Art der Auswahl bzw. Identifikation durch einen Benutzer) wird dieses automatisch als sekundäre Verordnung anstelle des äquivalenten, nicht verblisterbaren Medikaments in den Verordnungsplan eingefügt.

Auf der Medikamentenebene wird bei einem gegebenen Medikament vorzugsweise die dazugehörige PZN mit dem Verfügbarkeitsdatensatz (Positivliste) des Blisterzentrums verglichen. Die Positivliste kann dabei im System vorhanden sein, oder dynamisch angebunden werden, z.B. über einen Webservice aus dem Blisterzentrum oder einen SOA (Service-orientierte Architektur)-basierten Webservice. Für den Fall, dass die gegebene PZN in der Positivliste des Blisterzentrums vorhanden ist, wird als Ergebnis der erste Wert (z.B. "YES") zurückgegeben.

Bei der individuellen Verblisterung von Arzneimitteln für einen Patienten werden verschiedene Medikamente (z.B. unterschiedlicher Hersteller und/oder für verschiedene Verordnungen) gemeinsam pro Dosierungszeitpunkt verpackt. Die Kapazität der bei der automatisierten Verblisterung eingesetzten Maschinen ist beispielsweise auf einige Hundert verschiedene Medikamente begrenzt. Vorzugsweise wird für eine oder mehrere Verblisterungsmaschinen bzw. Verblisterungsanlagen bzw. Verblisterungseinheiten jeweils ein Eignungsdatensatz bzw. Verfügbarkeitsdatensatz bereitgestellt. In einer bevorzugten Ausführungsform wird der zumindest eine Verfügbarkeitsdatensatz in Form einer Positivliste bereitgestellt. Besonders bevorzugt wird der zumindest eine Verfügbarkeitsdatensatz von einem bzw. über einen ersten Web-Service bereitsgestellt.

Vorzugsweise basiert das erfindungsgemäße Verfahren auf mehreren Datenquellen, aus denen Informationen dynamisch zusammengeführt werden. Insbesondere steht am Beginn eine Identifikationsnummer für ein pharmazeutisches Erzeugnis, zum Beispiel eine Pharmazentralnummer (PZN), wie sie in Deutschland üblich ist. Eine bevorzugte Implementierung könnte alternativ oder zusätzlich auch andere Identifikationsnummern wie z.B. M2 unterstützen.

Nachfolgend ist ein beispielhafter Pseudocode zur Darstellung bzw. Implementierung eines erfindungsgemäßen Verfahrens in einer bevorzugten Ausführungsform dargestellt.

```
 public BlisterCheckResult checkDrugForBlisterEligibility(String pzn){
      if(isPZNlnPositiveList(PZNUtil.getSafePZN(pzn))){
            return new BlisterCheckResult(pzn:pzn, blisterEligibility:
            BlisterEligibility.YES
      }
      else if(isAlternativelnPositiveList(PZNUtil.getSafePZN(pzn))){
            return new BlisterCheckResult(pzn:pzn, blisterEligibility:
            BlisterEligibility.MAYBE, alternativeslnPositivList:
            equivalentMap[PZNUtil.getSafePZN(pzn)])
      }
      else{
            return new BlisterCheckResult(pzn:pzn, blisterEligibility:
      BlisterEligibility.NO)
      }
 }
```

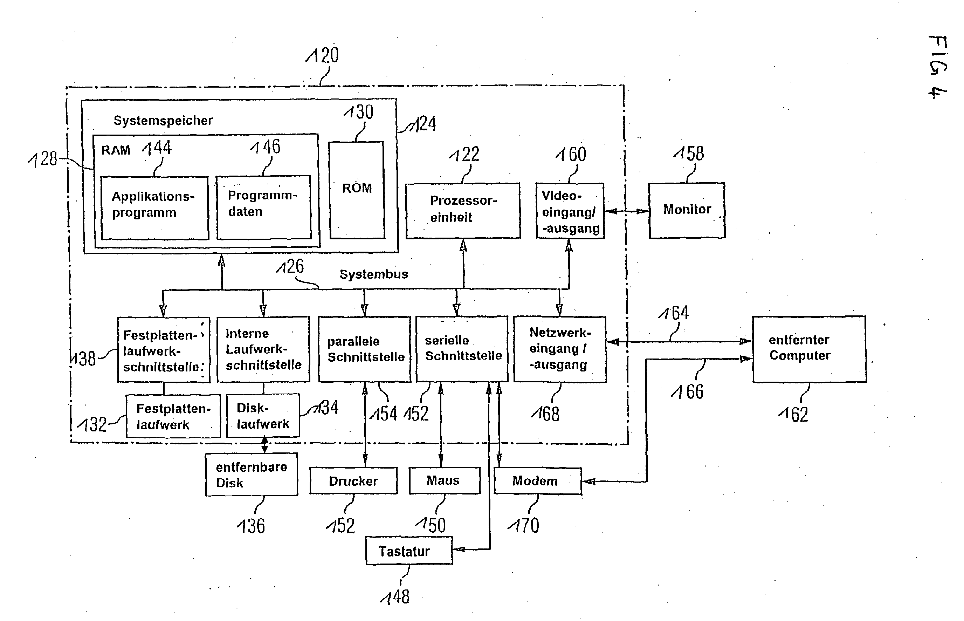

Bezugnehmend auf **Fig. 4** wird ein beispielhaftes System zum Implementieren der Erfindung beschrieben. Ein beispielhaftes System umfaßt eine universelle Rechnereinrichtung in der Form einer herkömmlichen Rechnerumgebung 120 z.B. ein "personal computer" (PC) 120, mit einer Prozessoreinheit 122, einem Systemspeicher 124 und einem Systembus 126, welcher eine Vielzahl von Systemkomponenten, unter anderem den Systemspeicher 124 und die Prozessoreinheit 122 verbindet. Die Prozessoreinheit 122 kann arithmetische, logische und/oder Kontrolloperationen durchführen, indem auf den Systemspeicher 124 zugegriffen wird. Der Systemspeicher 124 kann Informationen und/oder Instruktionen zur Verwendung in Kombination mit der Prozessoreinheit 122 speichern. Der Systemspeicher 124 kann flüchtige und nichtflüchtige Speicher, beispielsweise "random access memory" (RAM) 128 und "Nur-Lesespeicher" (ROM) 130 beinhalten. Ein Grund-Eingabe-Ausgabe-Sytem (BIOS), das die grundlegenden Routinen enthält, welche helfen, Informationen zwischen den Elementen innerhalb des PCs 120, beispielsweise während des Hochfahrens, zu transferieren, kann in dem ROM 130 gespeichert sein. Der Systembus 126 kann eine von vielen Busstrukturen sein, unter anderem ein Speicherbus oder ein Speichercontroller, ein peripherer Bus und ein lokaler Bus, welcher eine bestimmte Busarchitektur aus einer Vielzahl von Busarchitekturen verwendet.

Der PC 120 kann weiterhin ein Festplattenlaufwerk 132 zum Lesen oder Schreiben einer Festplatte (nicht gezeigt) aufweisen und ein externes Disklaufwerk 134 zum Lesen oder Schreiben einer entfernbaren Disk 136 bzw. eines entfernbaren Datenträgers. Die entfernbare Disk kann eine magnetische Disk bzw. eine magnetische Diskette für ein magnetisches Disklaufwerk bzw. Diskettenlaufwerk oder eine optische Diskette wie z.B. eine CD-ROM für ein optisches Disklaufwerk sein. Das Festplattenlaufwerk 132 und das externe Disklaufwerk 134 sind jeweils mit dem Systembus 126 über eine Festplattenlaufwerkschnittstelle 138 und eine externe Disklaufwerkschnittstelle 140 verbunden. Die Laufwerke und die zugeordneten computerlesbaren Medien stellen einen nichtflüchtigen Speicher computerlesbarer Instruktionen, Datenstrukturen, Programm-Modulen und anderer Daten für den PC 120 zur Verfügung. Die Datenstrukturen können die relevanten Daten zum Implementieren eines wie oben beschriebenen Verfahrens aufweisen. Obwohl die beispielshaft beschriebene Umgebung eine Festplatte (nicht gezeigt) und eine externe Disk 142 verwendet, ist für den Fachmann offensichtlich, daß andere Typen computerlesbarer Medien, welche computerzugreifbare Daten speichern können, in der beispielhaften Arbeitsumgebung verwendet werden können, wie z.B. magnetische Kassetten, Flash-Memory Karten, digitale Videodisketten, Random-Access-Speicher, Nur-Lesespeicher, usw..

Eine Vielzahl von Programm-Modulen, insbesondere ein Betriebssystem (nicht gezeigt) ein oder mehrere Applikationsprogramme 144, oder Programm-Module (nicht gezeigt) und Programmdaten 146, können auf der Festplatte, der externen Disk 142, dem ROM 130 oder dem RAM 128 gespeichert werden. Die Applikationsprogramme können zumindest einen Teil der Funktionalität, wie in Fig. 1 gezeigt, umfassen.

Ein Benutzer kann Kommandos und Information, wie oben beschrieben, in den PC 120 anhand von Eingabevorrichtungen, wie z.B. einer Tastatur bzw. eines Keyboards 148 und einer Computermaus 150 eingeben. Andere Eingabevorrichtungen (nicht gezeigt) können ein Mikrofon und/andere Sensoren, einen Joystick, ein Spielpolster bzw. -kissen, einen Scanner oder ähnliches umfassen. Diese oder andere Eingabevorrichtungen können mit der Prozessoreinheit 122 anhand einer seriellen Schnittstelle 152 verbunden sein, welche mit dem System 126 gekoppelt ist, oder können anhand anderer Schnittstellen, wie z.B. einer parallelen Schnittstelle 154, eines Spieleports oder eines universellen seriellen Busses (USB) verbunden sein. Weiterhin kann Information mit einem Drucker 156 gedruckt werden. Der Drucker 156 und andere parallele Eingabe/Ausgabevorrichtungen können mit der Prozessoreinheit 122 durch die parallele Schnittstelle 154 verbunden sein. Ein Monitor 158 oder andere Arten von Anzeigevorrichtung(en) ist/sind mit dem Systembus 126 mittels einer Schnittstelle, wie z.B. eines Videoeingang/-ausgangs 160 verbunden. Zusätzlich zu dem Monitor kann die Rechnerumgebung 120 andere periphere Ausgabevorrichtungen (nicht gezeigt) wie z.B. Lautsprecher oder akustische Ausgänge umfassen.

Die Rechnerumgebung 120 kann mit anderen elektronischen Vorrichtungen z.B. einem Computer, einem Schnurtelefon, einem schnurlosen Telefon, einem persönlichen digitalen Assistenten (PDA), einem Fernseher oder ähnlichem kommunizieren. Um zu kommunizieren, kann die Rechnerumgebung 120 in einer vernetzten Umgebung arbeiten, wobei Verbindungen zu einem oder mehreren elektronischen Vorrichtungen verwendet werden. Fig. 4 stellt die mit einem "remote computer" bzw. entfernten Computer 162 vernetzte Rechnerumgebung dar. Der entfernte Computer 162 kann eine andere Rechnerumgebung, wie z.B. ein Server, ein Router, ein Netzwerk-PC, eine gleichwertige bzw. "peer" Vorrichtung oder andere gewöhnliche Netzwerkknoten sein und kann viele oder alle der hinsichtlich der Rechnerumgebung 120 oben beschriebenen Elemente umfassen. Die logischen Verbindungen, wie sie in Fig. 4 dargestellt sind, umfassen ein "local area network" (LAN) 164 und ein "wide are network" (WAN) 166. Solche Netzwerkumgebungen sind alltäglich in Büros, firmenweiten Computernetzwerken, Intranetzen und dem Internet.

Wenn eine Rechnerumgebung 120 in einer LAN-Netzwerkumgebung verwendet wird, kann die Rechnerumgebung 120 mit dem LAN 164 durch einen Netzwerkeingang/-ausgang 168 verbunden sein. Wenn die Rechnerumgebung 120 in einer WAN-Netzwerkumgebung verwendet wird, kann die Rechnerumgebung 120 ein Modem 170 oder andere Mittel zum Herstellen einer Kommunikation über das WAN 166 umfassen. Das Modem 170, welches intern und extern bezüglich der Rechnerumgebung 120 sein kann, ist mit dem Systembus 126 mittels der seriellen Schnittstelle 152 verbunden. In der Netzwerkumgebung können Programm-Module, welche relativ zu der Rechnerumgebung 120 dargestellt sind, oder Abschnitte davon in einer entfernten Speichereinrichtung gespeichert sein, welche an oder von einem entfernten Computer 162 zugreifbar bzw. systemeigen sind. Weiterhin können andere Daten, welche für das oben beschriebene Verfahren bzw. System relevant sind, auf oder von dem entfernten Computer 162 zugreifbar vorliegen.

### Bezugszeichenliste

- 10: Verordnung
- 12: Eingabefeld
- 14: "Löschen"-Feld
- 16: Editierfeld
- 18: Verblisterbarkeitsindikator
- 30: Verordnungsplan
- 30a-c: Verordnungen
- 32: Verfügbarkeitsdatensatz
- 34a-c: Verblisterbarkeitsparameter
- 36: Äquivalenzdatenbank
- 38: Äquivalenzdatensatz
- 120: Rechnerumgebung
- 122: Prozessoreinheit
- 124: Systemspeicher
- 126: Systembus
- 128: random access memory (RAM)
- 130: Nur-Lesespeicher (ROM)
- 132: Festplattenlaufwerk
- 134: Disklaufwerk
- 136: entfernbare Disk
- 138: Festplattenlaufwerkschnittstelle
- 140: Disklaufwerkschnittstelle
- 142: externe Disk
- 144: Applikationsprogramm
- 146: Programmdaten
- 148: Tastatur
- 150: Computermaus
- 152: serielle Schnittstelle
- 154: parallele Schnittstelle
- 156: Drucker
- 158: Monitor
- 160: Videoeingang/ -ausgang
- 162: entfernter Computer
- 164: "local area network" (LAN)
- 166: "wide are network" (WAN)
- 168: Netzwerkeingang/ -ausgang

## Patentansprüche

1. Computer-implementiertes Verfahren zur Erstellung und/oder Optimierung eines medizinischen Verordnungsplans für zumindest einen Patienten, umfassend:
- Erfassen von primären Verordnungsdaten für den zumindest einen Patienten, wobei die primären Verordnungsdaten für zumindest ein Medikament eine primäre Arzneimittelidentifikation umfassen;
- Festlegen eines Verblisterbarkeitsparameters für das zumindest eine Medikament, wobei das Festlegen des Verblisterbarkeitsparameters umfasst:
-- eine primäre Verfügbarkeitsprüfung zum Auswerten eines Verfügbarkeitsdatensatzes dahingehend, ob die primäre Arzneimittelidentifikation im Verfügbarkeitsdatensatz enthalten ist; und in Abhängigkeit vom Ergebnis der primären Verfügbarkeitsprüfung:
-- Zuweisen eines ersten Wertes zu dem Verblisterbarkeitsparameter, falls die primäre Arzneimittelidentifikation im Verfügbarkeitsdatensatz enthalten ist; und
-- eine Äquivalenzprüfung, falls die primäre Arzneimittelidentifikation im Verfügbarkeitsdatensatz nicht enthalten ist, wobei die Äquivalenzprüfung umfasst:
--- Ermitteln eines Äquivalenzdatensatzes aus einer Äquivalenzdatenbank derart, dass der Äquivalenzdatensatz Arzneimittelidentifikationen zumindest teilweise umfasst, die der primären Arzneimittelidentifikation durch die Äquivalenzdatenbank zugeordnet werden;
--- eine sekundäre Verfügbarkeitsprüfung zum Auswerten des Verfügbarkeitsdatensatzen dahingehend, ob zumindest eine Arzneimittelidentifikation des Äquivalenzdatensatzes im Verfügbarkeitsdatensatz enthalten ist; und
in Abhängigkeit vom Ergebnis der sekundären Verfügbarkeitsprüfung:
--- Zuweisen eines zweiten Wertes zu dem Verblisterbarkeitsparameter, falls zumindest eine Arzneimittelidentifikation des Äquivalenzdatensatzes im Verfügbarkeitsdatensatz enthalten ist; und
--- Zuweisen eines dritten Wertes zu dem Verblisterbarkeitsparameter, falls keine Arzneimittelidentifikation des Äquivalenzdatensatzes im Verfügbarkeitsdatensatz enthalten ist; und
- Ausgeben eines Verblisterbarkeitsindikators in Bezug auf die primäre Arzneimittelidentifikation und in Abhängigkeit vom zugewiesenen Wert des Verblisterbarkeitsparameters.

2. Verfahren nach Anspruch 1, wobei die sekundäre Verfügbarkeitsprüfung ein Erstellen eines Austauschdatensatzes umfasst, welcher sekundäre Arzneimittelidentifikationen festlegt, die sowohl im Äquivalenzdatensatz als auch im Verfügbarkeitsdatensatz enthalten sind, und wobei das Verfahren im Fall, dass dem Verblisterbarkeitsparameter in Bezug auf das zumindest eine Medikament der zweite Wert zugewiesen wurde, umfasst:
- Erfassen einer Austauschabfrage durch eine Benutzereingabe; und
- Ausgeben von Repräsentationen der sekundären Arzneimittelidentifikationen.

3. Verfahren nach Anspruch 2, wobei das Verfahren außerdem umfasst:
- Erfassen einer Auswahl einer sekundären Arzneimittelidentifikation aus dem Austauschdatensatz; und
- Erstellen von sekundären Verordnungsdaten aus den primären Verordnungsdaten umfassend ein Ersetzen der primären Arzneimittelidentifikation durch die sekundäre Arzneimittelidentifikation.

4. Verfahren nach Anspruch 3, wobei die primären Verordnungsdaten für das zumindest eine Medikament primäre Dosierungsdaten umfassen und wobei das Erstellen der sekundären Verordnungsdaten umfasst:
- Ermitteln von sekundären Dosierungsdaten für ein durch die sekundäre Arzneimittelidentifikation festgelegtes Austauschmedikament aus der Äquivalenzdatenbank in Abhängigkeit von den primären Dosierungsdaten; und
- Ersetzen der primären Dosierungsdaten durch die sekundären Dosierungsdaten.

5. Verfahren nach einem der vorangegangenen Ansprüche, umfassend:
- Erfassen einer Patientenidentifikation für den zumindest einen Patienten; und
- Abrufen von gespeicherten Patientendaten aus einer Patientendatenbank in Abhängigkeit von der Patientenidentifikation.

6. Verfahren nach Anspruch 5, wobei das Erfassen von primären Verordnungsdaten für den zumindest einen Patienten ein Abrufen der primären Verordnungsdaten für den zumindest einen Patienten aus der Patientendatenbank in Abhängigkeit von der Patientenidentifikation umfasst.

7. Verfahren nach Anspruch 5 oder 6, umfassend ein Bereitstellen einer Kompatibilitätsdatenbank, in der einer Vielzahl von Arzneimittelidentifikationen eine oder mehrere andere Arzneimittelidentifikationen als inkompatibel zugeordnet werden,
wobei die gespeicherten Patientendaten konsolidierte Verordnungsdaten umfassen, welche jeweils zumindest eine konsolidierte Arzneimittelidentifikation umfassen und
wobei die Äquivalenzprüfung eine Kompatibilitätsprüfung derart umfasst, dass der Äquivalenzdatensatz keine Arzneimittelidentifikationen umfasst, die zumindest einer der konsolidierten Arzneimittelidentifikationenen durch die Kompatibilitätsdatenbank als inkompatibel zugeordnet werden.

8. Verfahren nach einem der Ansprüche 5 bis 7, wobei die gespeicherten Patientendaten einen Akzeptanzdatensatz des zumindest einen Patienten umfassen, welcher eine oder mehrere Arzneimittelidentifikationen als von dem zumindest einen Patienten nicht akzeptiert festlegt, und wobei die Äquivalenzprüfung eine Akzeptanzprüfung derart umfasst, dass der Äquivalenzdatensatz keine Arzneimittelidentifikation umfasst, die vom Akzeptanzdatensatz als von dem zumindest einen Patienten nicht akzeptiert festgelegt ist.

9. Verfahren nach einem der vorangegangen Ansprüche, wobei die primären Verordnungsdaten für eine Vielzahl von Medikamenten jeweils eine primäre Arzneimittelidentifikationen umfassen, und wobei das Festlegen eines Verblisterbarkeitsparameters für eine Vielzahl dieser Medikamente erfolgt.

10. Verfahren nach Anspruch 9, umfassend ein Zuweisen eines Effizienzwerts zu dem Verfügbarkeitsdatensatz, wobei das Zuweisen des Effizienzwerts umfasst:
- Bereitstellen einer Effizienzwertrelation, die jedem Wert des Verblisterbarkeitsparameters einen Effizienzgewichtungswert zuordnet;
- Ermitteln des Effizienzwerts in Abhängikeit von den Effizienzgewichtungswerten aller zu diesem Verfügbarkeitsdatensatz für die einzelnen Medikamente festgelegten Verblisterbarkeitsparameter.

11. Verfahren nach Anspruch 10, umfassend ein
- Bereitstellen einer Vielzahl von Verfügbarkeitsdatensätzen, denen jeweils ein Auftragsdatensatz zugeordnet ist, wobei das Zuweisen eines Effizienzwerts für jeden der Verfügbarkeitsdatensätze erfolgt;
- Ermitteln eines optimalen Verfügbarkeitsdatensatzes aus der Vielzahl von Verfügbarkeitsdatensätzen auf Basis der ihnen zugewiesenen Effizienzwerte; und
- Zuordnen des dem optimalen Verfügbarkeitsdatensatz zugeordneten Auftragsdatensatzes zu Patientendaten des zumindest einen Patienten.

12. Computer-implementiertes Verfahren zur individuellen Verblisterung von Medikamenten für zumindest einen Patienten, umfassend:
- ein Verfahren zur Erstellung und/oder Optimierung eines medizinischen Verordnungsplans nach Anspruch 3, 4 oder einem der Ansprüche 5 bis 11, soweit er auf Anspruch 3 rückbezogen ist;
- Ermitteln eines dem Patienten zugeordneten Auftragsdatensatzes;
- Ermitteln von dem Patienten zugeordneten Versorgungsintervalldaten;
- Übermitteln der sekundären Verordnungsdaten zusammen mit den Versorgungsintervalldaten an eine durch den Auftragsdatensatz festgelegte, automatisch gesteuerte Verblisterungsanlage; und
- automatisches Verblistern von Medikamenten gemäß den sekundären Verordnungsdaten für einen in den Versorgungsintervalldaten festgelegten Versorgungszeitraum.

13. Computersystem zur Erstellung und/oder Optimierung eines medizinischen Verordnungsplans für zumindest einen Patienten, umfassend:
- eine Eingabeschnittstelle zum Erfassen von primären Verordnungsdaten für den zumindest einen Patienten, wobei die primären Verordnungsdaten für zumindest ein Medikament eine primäre Arzneimittelidentifikation umfassen;
- ein Analysemodul, welches ausgelegt ist zum Festlegen eines Verblisterbarkeitsparameters für das zumindest eine Medikament, wobei das Festlegen des Verblisterbarkeitsparameters umfasst:
-- eine primäre Verfügbarkeitsprüfung zum Auswerten eines Verfügbarkeitsdatensatzes dahingehend, ob die primäre Arzneimittelidentifikation im Verfügbarkeitsdatensatz enthalten ist; und in Abhängigkeit vom Ergebnis der primären Verfügbarkeitsprüfung:
-- Zuweisen eines ersten Wertes zu dem Verblisterbarkeitsparameter, falls die primäre Arzneimittelidentifikation im Verfügbarkeitsdatensatz enthalten ist; und
-- eine Äquivalenzprüfung, falls die primäre Arzneimittelidentifikation im Verfügbarkeitsdatensatz nicht enthalten sind, wobei die Äquivalenzprüfung umfasst:
--- Ermitteln eines Äquivalenzdatensatzes aus einer Äquivalenzdatenbank derart, dass der Äquivalenzdatensatz Arzneimittelidentifikationen zumindest teilweise umfasst, die der primären Arzneimittelidentifikation durch die Äquivalenzdatenbank zugeordnet werden;
--- eine sekundäre Verfügbarkeitsprüfung zum Auswerten des Verfügbarkeitsdatensatzen dahingehend, ob zumindest eine Arzneimittelidentifikation des Äquivalenzdatensatzes im Verfügbarkeitsdatensatz enthalten ist; und
in Abhängigkeit vom Ergebnis der sekundären Verfügbarkeitsprüfung:
--- Zuweisen eines zweiten Wertes zu dem Verblisterbarkeitsparameter, falls zumindest eine Arzneimittelidentifikation des Äquivalenzdatensatzes im Verfügbarkeitsdatensatz enthalten ist; und
--- Zuweisen eines dritten Wertes zu dem Verblisterbarkeitsparameter, falls keine Arzneimittelidentifikation des Äquivalenzdatensatzes im Verfügbarkeitsdatensatz enthalten ist; und
- eines Ausgabeeinheit zum Ausgeben eines Verblisterbarkeitsindikators in Bezug auf die primäre Arzneimittelidentifikation und in Abhängigkeit vom zugewiesenen Wert des Verblisterbarkeitsparameters.

14. Computersystem nach Anspruch 13, welches ausgelegt ist, ein Verfahren nach einem der Ansprüche 1 bis 12 auszuführen.

15. Computerprogrammprodukt, welcher Programmcode umfasst, der, wenn er geladen wird auf einem Computer, zur Ausführung eines Verfahrens nach einem der Ansprüche 1 bis 12 geeignet ist.
